# EUROPEAN PATENT SPECIFICATION

(11) **EP 3 827 814 B1**
(45) Date of publication and mention of the grant of the patent: **17.12.2025**
(21) Application number: 20209808.3
(22) Date of filing: 25.11.2020
(51) Int. Cl.: A61K 9/14, A61K 47/26, A61K 47/38, A61K 9/00, A61K 9/08

(54) **OPHTHALMIC FORMULATIONS**
OPHTHALMISCHE FORMULIERUNGEN
FORMULATIONS OPHTALMIQUES

(30) Priority: 25.11.2019 IT 201900022029
(43) Date of publication of application: 02.06.2021
(73) Proprietor: Dompé farmaceutici S.p.A., 20122 Milano (IT)
(72) Inventor: MANTELLI, Flavio, 67100 L'Aquila (IT); GENTILE, Marco Maria, 67100 L'Aquila (IT); DRAGANI, Maria Concetta, 67100 L'Aquila (IT)
(74) Representative: Dompé farmaceutici Spa

(56) References cited:
- WO-A1-2020/210248
- CN-A- 101 181 279
- US-A1- 2019 224 120

## Description

### Background of the invention

The corneal epithelium is the cornea's outermost portion. It measures about 50 microns and is composed of five to seven layers of very regularly arranged, non-keratinised squamous epithelial cells. Its external surface is covered by a thin continuous coating of tears while the internal surface is in contact with the Bowman's layer which connects the epithelium to the corneal stroma.

The corneal epithelium acts as a protecting barrier for the cornea, contributes to maintenance of a constant level of hydration in the stroma and serves as an optical interface. In particular, the barrier function of the epithelium is directed against physical trauma, pathogens and chemicals, threats to the underlying stroma that may lose its transparency if damaged.

The epithelial cells are characterized by a very fast turnover: squamous epithelial cells are continuously shed into the tear pool and simultaneously replenished by cells moving centrally from the limbus and anteriorly from the basal layers of the epithelium. This turnover is further accelerated following an injury so that lesions that involve the epithelium usually heal spontaneously and rapidly.

However, in some pathological conditions, the above equilibrium is compromised resulting in failure of the mechanisms promoting corneal reepithelization. In these situations, the normal regeneration processes of the epithelium are not effective in repairing a wound, leading to focal areas of epithelial loss referred to as corneal epithelial defects.

A variety of etiological factors may cause corneal epithelial defects such as, for example, abnormalities in the lids or in the tear film, inflammatory diseases, mechanical traumas, epithelial stem cell deficiency, surgery or various ocular surface disorders, including dry eye syndrome, neurotrophic keratopathy, recurrent erosion syndrome, and superior limbic keratoconjunctivitis such as keratoconjunctivitis sicca. Corneal epithelial defects are one of the most commonly seen ocular pathologies in the general patient population.

The area of epithelial loss is usually visualized by using a fluorescein dye, which is instilled either as drops (mixed with a topical anaesthetic) or via a fluorescein impregnated paper strip after the instillation of a topical anaesthetic. The dye is visualized using a cobalt-blue filter which causes the dye to fluoresce a bright green color. Fluorescein does not stain intact corneal epithelium but does stain corneal stroma, thus demarcating the area where epithelial loss has occurred.

Several therapeutic procedures are used to date for the management of corneal epithelial defects.

Topical antibiotics are usually administered to all the patients, independently from the cause of the lesion, as a prophylactic treatment against infections. Other treatments are specifically selected on the basis of the clinical condition of the epithelium and to address the underlying etiology.

These treatments may include, for example, lubrication and protection of the ocular surface from desiccation, bandage contact lenses, pressure patching, tarsorrhaphy, oral tetracyclines and corticosteroids.

For example, lubricating artificial tears containing water soluble polymers such for example hydroxyethylcellulose, methylcellulose, carboxymethylcellulose, hyaluronic acid, polyethylene glycol, propylene glycol, glycerine, polysorbate, polyvinyl alcohol, polyvinylpyrrolidone and polyacrylic acid may be used. These polymers have the ability to absorb water, thereby expanding the thickness of the tear film on the surface of the eye, thereby protecting the cornea and reducing friction and irritational discomfort.

The common goal of the different treatments is to cover and protect the corneal epithelial defect in order to assist proliferation of limbal stem cells and migration of mature corneal epithelial cells to heal the epithelial defects.

Also, trehalose has been recently introduced as a treatment in dry eye patients in order to prevent corneal epithelial defects. It is a natural disaccharide and acts by protecting epithelial corneal cells from desiccation and high osmolarity.

Unfortunately, in a considerable number of patients, corneal epithelial defects are not responsive to the standard therapeutic treatments available and the above therapeutic strategies show little efficacy.

The patent WO 2020/210248 A1 discloses a composition for treating ocular conditions comprising MSC secretome, mannitol, trehalose dihydrate, hypromellose and PEG.

The patent US 2019/224120 A1 discloses an ophthalmic composition comprising trehalose, one or more non-ionic surfactants, a polyethylene glycol, mannitol and carboxymethyl cellulose.

CN 101 181 279 A discloses artificial tears containing trehalose and Hypromellose. There is therefore a need in the art for the development of more effective ophthalmic formulations that can be used in the treatment of corneal epithelial defects.

### Summary of the invention

It has now been surprisingly found that a combination of trehalose, mannitol and hydroxypropylmethyl cellulose is particularly effective in inducing healing of corneal epithelial defects when administered topically to the eye.

In particular, as will be demonstrated in the experimental section, the present inventors have found that the combination of the above components results in a synergistic effect on the epithelial healing process.

Furthermore, the inventors have found that the healing-inducing effect observed is independent from the lubricant properties of the formulation. In fact, formulations having lubricant activity similar to that of the formulations according to the invention but not containing the above combination of components do not show an equivalent ability to induce healing.

Accordingly, one object of the present invention is an ophthalmic formulation comprising trehalose, mannitol and hydroxypropylmethyl cellulose.

Another object of the present invention is an ophthalmic formulation according to the first object of the invention for use in the prevention and/or treatment of an epithelial corneal defect.

### Definitions

The term "ophthalmic" referred to a formulation or solution refers to a formulation or solution suitable to be administered to the eye.

The term "ophthalmically acceptable excipients" refers to excipients that are suitable to be included in an ophthalmic formulation. The term "excipient" refers to substances present in a formulation not having any therapeutic activity.

The term "buffering agent" refers to compounds, usually weak acids or bases, that are able to maintain the acidity (pH) of a solution near a chosen value after the addition of another acid or base.

The term "non-ionic surfactants" as used herein refers to surfactants having an uncharged hydrophilic group, that does not ionize at any pH value.

Examples of non-ionic surfactant comprise poloxamers, polysorbates, cyclodextrins, alkylaryl polyethers, polyoxyethyleneglycol alkyl ethers, tyloxapol, and polyoxyls.

### Figures

Figure 1 shows the percentage of the damaged area induced by subcutaneous injections of scopolamine hydrobromide, in mice not treated (Control), treated with commercial lubricating eye drops (Old) or treated with the ophthalmic formulation n.2 in Example 1 (New), measured as described in Example 2.
Figure 2 shows the mean number of cells per microscopic field stained by trypan blue dye in a test carried out on the immortalized human corneal epithelial cell line HCEpiC exposed to a desiccating stress and treated with a Saline Buffer or different compositions, measured as described in Example 3.

### Description

As demonstrated in the experimental section, the inventors have found that the administration of a formulation containing trehalose, mannitol and hydroxypropylmethyl cellulose induces healing of corneal epithelium defects. In particular, as will be described in the experimental section, when the above three components are combined a synergistic effect on the healing process is observed. Accordingly, a first object of the present invention is an ophthalmic formulation comprising trehalose, mannitol and hydroxypropylmethyl cellulose.

Preferably, hydroxypropylmethyl cellulose has a molecular weight such as to provide a 2% solution in water with a viscosity between 3800 and 4300 mPas, preferably 4,000 mPas.

Preferably, the ophthalmic formulation according to the invention contains only one cellulose ether.

The ophthalmic formulation according to the invention comprises trehalose, mannitol and hydroxypropylmethyl cellulose.

Preferably, the ophthalmic formulation according to the invention does not comprise any nonionic surfactant.

More preferably, said ophthalmic formulation does not comprise any surfactant.

Preferably, the ophthalmic formulation according to the invention further comprises an antioxidant compound. The inventors have found that the presence of an antioxidant compound further increases the efficacy of the formulation in inducing corneal epithelial healing.

Any antioxidant suitable for ophthalmic use may be used in the formulation according to the invention.

Preferably, said antioxidant compound is selected from L-methionine and cysteine. Preferably, the ophthalmic formulation according to the invention further comprises buffering agents. Preferably, said buffering agent are suitable to maintain the pH of a solution between 6.5 and 8, more preferably between 6.8 and 7.5, even more preferably between 7 and 7.4. Preferably, said buffering agents are selected from sodium phosphate, phosphate/citrate, tris phosphate/borate, amminoacid agents or combinations thereof, preferably sodium phosphate buffer.

Preferably, the ophthalmic formulation according to the invention further comprises a polyethylene glycol.

Preferably, said polyethylene glycol has a molecular weight between 4000 and 8000 g/mol, more preferably between 5000 and 7000 g/mol, more preferably between 5500 and 6500 g/mol, even more preferably of 6000 g/mol.

According to a preferred embodiment, the ophthalmic formulation according to the present invention has a composition consisting of trehalose, mannitol, hydroxypropylmethyl cellulose and, optionally, one or more further components selected from the list consisting of antioxidant compounds, buffering agents, polyethylene glycols and ophthalmically acceptable excipients.

According to a particularly preferred embodiment, the ophthalmic formulation according to the present invention has a composition consisting of trehalose, mannitol, hydroxypropylmethyl cellulose and, optionally, one or more further components selected from the list consisting of antioxidant compounds, buffering agents and polyethylene glycols.

According to another particularly preferred embodiment, the ophthalmic formulation according to the present invention has a composition consisting of trehalose, mannitol, hydroxypropylmethyl cellulose and one or more antioxidant compounds, buffering agents and polyethylene glycols.

According to a further particularly preferred embodiment, the formulation has a composition consisting of trehalose, mannitol, hydroxypropylmethyl cellulose, buffering agents and a polyethylene glycol.

According to a further particularly preferred embodiment, the formulation has a composition consisting of trehalose, mannitol, hydroxypropylmethyl cellulose, and one or more antioxidant compounds, buffering agents and a polyethylene glycol

According to a further particularly preferred embodiment, the formulation has a composition consisting of trehalose, mannitol, hydroxypropylmethyl cellulose, buffering agents and a polyethylene glycol.

A preferred ophthalmic formulation according to the above preferred embodiments has a composition comprising, preferably consisting of, trehalose in an amount between 60 and 85 weight%, preferably between 70 and 80 weight%, mannitol in an amount between 10 and 30 weight%, preferably between 15 and 25 weight%, hydroxypropylmethyl cellulose, in an amount between 0.5 and 2.5 weight%, preferably between 1 and 2 weight% and, optionally, phosphate buffer in an amount between 3 and 10 weight%, preferably between 5 and 8 weight%, methionine in an amount between 0.005 and 0.02 weight%, preferably between 0.01 and 0.015 weight%, polyethylene glycol in an amount between 5 and 25 weight%, preferably between 10 and 20 weight%.

The ophthalmic formulation according to the present invention may be in form of a powder, for example a lyophilized powder, to be dissolved at the moment of use in a suitable aqueous diluent to form an extemporaneous ophthalmic solution, or may be a ready-to-use ophthalmic solution. Preferably, the above formulation is in form of a solution.

The pH of said extemporaneous or ready to use ophthalmic solution is preferably between 6.5 and 8, more preferably between 6.8 and 7.5, even more preferably between 7 and 7.4.

Preferably, when the ophthalmic formulation is in form of an ophthalmic solution it contains said buffering agents.

The extemporaneous or ready to use ophthalmic solution according to the invention has an osmolarity preferably between 200 and 380 mOsmol/kg, more preferably between 250 and 330 mOsmol/kg, even more preferably between 280 and 320 mosm/Kg.

Preferably, trehalose is present in the ophthalmic solution according to the invention in a concentration comprised between 40 and 60 mg/ml, more preferably between 45 and 55 mg/ml, even more preferably between 48 and 52 mg/ml, even more preferably between 50 and 51 mg/ml.

Preferably, mannitol is present in the ophthalmic solution according to the invention in a concentration comprised between 8 and 20 mg/ml, more preferably between 10 and 16 mg/ml, even more preferably between 12 and 14 mg/ml, even more preferably between 13 and 13.5 mg/ml.

Preferably, the concentration of hydroxypropylmethyl cellulose in the ophthalmic solution according to the invention is such as to obtain a kinematic viscosity of about 1-25 cSt determined at 25°C using a capillary viscometer.

Preferably, the total amount of hydroxypropylmethyl cellulose in the ophthalmic solution according to the invention is between 0.4 and 2 mg/ml, preferably between 0.8 and 1.6 mg/ml, even more preferably between 1 and 1.3 mg/ml.

Preferably, the concentration of the buffering agent in the ophthalmic solution according to the present invention is between 2 and 6 mg/ml, preferably between 4 and 5 mg/ml.

Preferably, the concentration of polyethylene glycol in the ophthalmic solution according to the invention, when present, is comprised between 6 and 14 mg/ml, more preferably between 8 and 13 mg/ml, even more preferably between 9 and 12 mg/ml, even more preferably between 10 and 11 mg/ml.

Preferably, the concentration of said anti-oxidant in the ophthalmic solution according to the present invention, when present, is between 0.004 and 0.02 mg/ml, more preferably between 0.008 to 0.012 mg/ml.

According to a preferred embodiment, when the ophthalmic formulation is in form of a powder it contains the above buffering agent in dry form.

According to an alternative embodiment, when the ophthalmic formulation is in form of a powder it does not contain any of the above buffering agent in dry form and buffering agents are present in the aqueous diluent in which the powder is dissolved to form the extemporaneous ophthalmic solution.

As will be demonstrated in the experimental section, the ophthalmic formulation according to the invention is effective in the prevention and/or healing of corneal epithelial defects.

Thus, a second object of the present invention is an ophthalmic formulation according to the first object of the invention for use in the prevention and/or treatment of corneal epithelial defects.

The present disclosure also discloses a method, not part of the invention, for prevention and/or treatment of corneal epithelial defects in a patient in need thereof, comprising i) identifying a subject affected by a corneal epithelia defect and ii) applying the ophthalmic solution according to the invention topically to the eyes of the subject.

Preferably, said corneal epithelial defects are corneal epithelial defects associated to impaired corneal sensitivity, dry eye, neurotrophic keratitis or surgery.

### EXPERIMENTAL SECTION

### Example 1

Ophthalmic solutions having the composition indicated in tables 1 and 2 below have been prepared by dissolving the ingredients in Water for Injection and adjusting the pH to 7-7.4.

**Table 1: formulation n.1.**

| Ingredients | Concentration (mg/ml) |
|---|---|
| Trehalose dihydrate | 50.4 |
| Mannitol | 13.1 |
| Phosphate buffer | 4.4 |
| HPMC 4000 mPa.s | 1.1 |
| PEG6000 | 10.7 |
| Water for Injection | qb to 1 ml |

**Table 2: formulation n. 2**

| Ingredients | Concentration (mg/ml) |
|---|---|
| Trehalose dihydrate | 50.4 |
| Mannitol | 13.1 |
| Phosphate buffer | 4.4 |
| HPMC 4000 mPa.s | 1.1 |
| PEG6000 | 10.7 |
| L-methionine | 0.01 |
| Water for Injection | qb to 1 ml |

Both formulations have an osmolarity between 280 and 320 mosm/Kg.

### Example 2

The ability of the ophthalmic formulation n. 2 above to prevent damage to the corneal epithelium was tested in a mouse model of scopolamine-induced dry eye, in comparison to a reference commercial lubricating eye drop formulation.

The reference formulation used contained sodium hyaluronate 0.18% as active ingredient and the excipients sodium chloride, potassium chloride, disodium phosphate, sodium citrate, magnesium chloride, calcium chloride in water for injections. The solution was hypotonic (150 mOsm/l) and adjusted to pH 7.3.

Mice (N=5 per group) were randomly assigned at baseline to three groups and they were all subjected to a subcutaneous injections protocol of 0.5 mg/0.2 ml scopolamine hydrobromide, as previously described, to induce dry eye and epithelial damage (Yeh S et al. Invest Ophthalmol Vis Sci, 2003).

The three groups consisted of a control group, which received no treatment, a group which was treated with ophthalmic formulation n. 2 and a group which was treated with the commercial lubricating eye drops formulation described above.

In details, the animals of the treated groups received ten microliters of the test formulations instilled three times a day in both eyes from day 1 after the first scopolamine injection until day 25. The control group did not receive any eye drop treatment. The treatments were encoded, and the group allocation was blinded to the technician administering the treatment, and to the researcher assessing the outcome of the experiment. Group identification was uncovered at the end of the analysis.

*In vivo* fluorescein staining was performed throughout the study until completion at day 25, digital images were obtained under slit lamp with a cobalt blue light at 10X magnification and analyzed in a semi-automatic manner for fluorescein staining using ImageJ software.

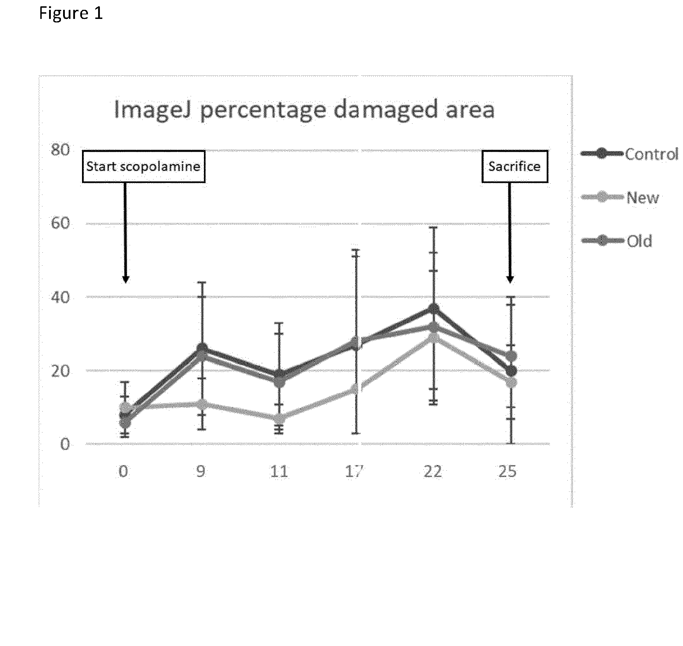

As shown in Figure 1, in the time frame of this study, treatment with the reference formulation did not result in in any significant decrease, compared to the control, in the extent of the corneal wounding area induced by scopolamine. On the contrary, formulation n.2 significantly decreased the extent of epithelial damage compared to both control and the reference formulation.

These data demonstrate that the healing effect of the formulation according to the invention is due to a direct effect on the epithelial healing process and it is independent from the lubricant activity, as evident from the lack of effect for the commercial reference formulation. This is further suggested by the fact that the preventive effect is particularly evident at the early time points, in the first 17 days of the study, reaching statistical significance at day 9.

No significant difference was observed between the groups in other parameters that were assessed throughout the study, including tear secretion and leukocytic infiltration of the conjunctiva.

### Example 3

The ability of different compositions to prevent damage of corneal epithelial cells was tested in an immortalized human corneal epithelial cell line (HCEpiC, Innoprot, Spain).

In details, the cells were exposed to a desiccating stress as previously reported (Zebo Huang and Alan Tunnacliffe, J Physiol, 2004 Jul 1; 558(Pt 1): 181-191.): briefly, confluent cells were washed with PBS and, after liquid was carefully completely removed by aspiration, placed in humidity chambers and dried for 4 hours. The humidity in the chambers was maintained by saturated BaCl2 (90% relative humidity; RH), saturated CuSO4 (98% RH) or sterile water (100% RH) in an air-tight polypropylene box (in mm: 162 × 176 × 100; w × d × h) at 37°C.

The cells were then rehydrated for 2 hours at 37°C immediately prior to assay using a Dulbecco's modified Eagle's culture medium supplemented with 10% fetal bovine serum, to which the following were added:
- 4.4 mg/ml phosphate saline buffer (sample 1),
- 0.2% hyaluronic acid (sample 2),
- 50.4 mg/ml trehalose (sample 3),
- 50.4 mg/ml trehalose and 0.2% hyaluronic acid (sample 4),
- 50.4 mg/ml trehalose, 13.1 mg/ml mannitol and 1.1 mg/ml hydropropylmethylcellulose (HPMC 4000 mPa.s) (sample 5),
- 13.1 mg/ml mannitol and 1.1 mg/ml hydropropylmethylcellulose (HPMC 4000 mPa.s) (sample 6), or
- 13.1 mg/ml mannitol (sample 7), or 1.1 mg/ml hydropropylmethylcellulose (HPMC 4000 mPa.s) (Sample 8).

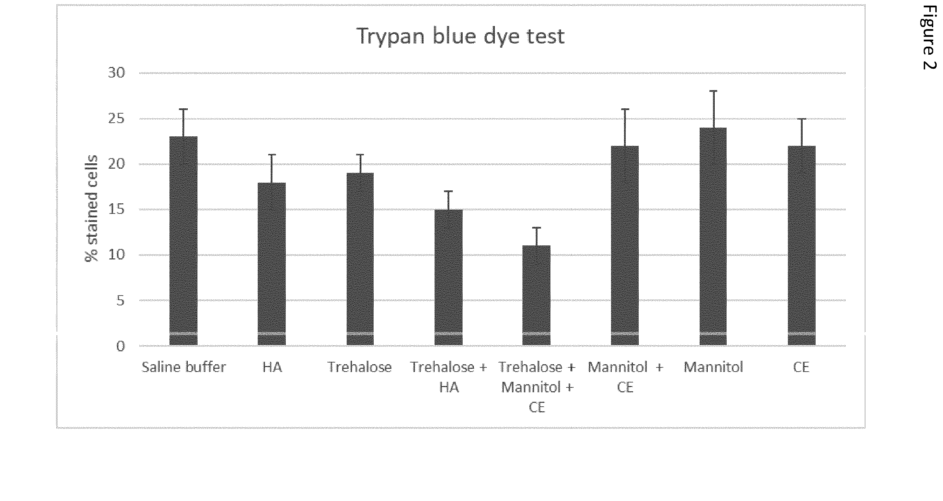

After incubation, the vitality of the cells in different samples 1 to 8 was evaluated with trypan blue assay as described in Aragona P et al, The Scientific World Journal, vol. 2014, Article ID 717835, 9 pages, 2014. In particular, trypan blue was used to measure the mean number of dead cells per microscopic field, which are reported in Figure 2.

As show in Figure 2, the combination of trehalose with mannitol and hydropropylmethylcellulose resulted in a significant lower number of stained cells per microscopic field compared to all other samples. The results clearly show that the combination of the three components achieve a decrease in the number of stained cells that is much higher than what expected on the basis of the results obtained by the single components or dual combinations thereof. This demonstrates the presence of a synergistic effect when the three components above are combined and confirm the healing effect on epithelial cells of such composition that has been observed *in vivo* in animal models.

## Claims

1. Ophthalmic formulation having a composition consisting of trehalose, mannitol, hydroxypropylmethyl cellulose and, optionally, one or more further components selected from the list consisting of antioxidant compounds, buffering agents, polyethylene glycols and ophthalmically acceptable excipients, wherein the ophthalmic formulation does not comprise any nonionic surfactant.

2. Ophthalmic formulation as claimed in claim 1, having a composition consisting of trehalose, mannitol, hydroxypropylmethyl cellulose and, optionally, one or more further components selected from the list consisting of antioxidant compounds, buffering agents and polyethylene glycols.

3. Ophthalmic formulation as claimed in claims 1 or 2, having a composition consisting of trehalose, mannitol, hydroxypropylmethyl cellulose, buffering agents and a polyethylene glycol.

4. Ophthalmic formulation as claimed in claim 1 or 2, wherein said antioxidant compounds are L-methionine and cysteine.

5. Ophthalmic formulation as claimed in claims 1 to 4, wherein said polyethylene glycols have a molecular weight between 4000 and 8000 g/mol, more preferably between 5000 and 7000 g/mol, more preferably between 5500 and 6500 g/mol, even more preferably of 6000 g/mol and/or said buffering agents are selected from sodium phosphate, phosphate/citrate, tris phosphate/borate, amminoacid agents or combinations thereof, preferably sodium phosphate buffer.

6. Ophthalmic formulation as claimed in claims 1 to 5, having a composition consisting of trehalose in an amount between 60 and 85 weight%, preferably between 70 and 80 weight%, mannitol in an amount between 10 and 30 weight%, preferably between 15 and 25 weight%, hydroxypropylmethyl cellulose in an amount between 0.5 and 2.5 weight%, preferably between 1 and 2 weight% and, optionally, phosphate buffer in an amount between 3 and 10 weight%, preferably between 5 and 8 weight%, methionine in an amount between 0.005 and 0.02 weight%, preferably between 0.01 and 0.015 weight%, polyethylene glycol in an amount between 5 and 25 weight%, preferably between 10 and 20 weight%.

7. Ophthalmic formulation as claimed in claims 1 to 6, in form of a powder, preferably a lyophilized powder.

8. Ophthalmic formulation as claimed in claims 1 to 6, in form of a solution.

9. Ophthalmic formulation as claimed in claim 8, wherein the pH of said solution is preferably between 6.5 and 8, more preferably between 6.8 and 7.5, even more preferably between 7 and 7.4 and/or the osmolarity of said solution is between 200 and 380 mOsmol/kg, preferably between 250 and 330 mOsmol/kg, more preferably between 280 and 320 mosm/Kg.

10. Ophthalmic formulation as claimed in claim 8 or 9, containing trehalose in a concentration comprised between 40 and 60 mg/ml, more preferably between 45 and 55 mg/ml, even more preferably between 48 and 52 mg/ml, even more preferably between 50 and 51 mg/ml, and/or mannitol in a concentration comprised between 8 and 20 mg/ml, more preferably between 10 and 16 mg/ml, even more preferably between 12 and 14 mg/ml, even more preferably between 13 and 13.5 mg/ml and/or hydroxypropylmethyl cellulose in a concentration between 0.4 and 2 mg/ml, preferably between 0.8 and 1.6 mg/ml, even more preferably between 1 and 1.3 mg/ml, and/or a buffering agent in a concentration between 2 and 6 mg/ml, preferably between 4 and 5 mg/ml, and/or an anti-oxidant in a concentration between 0.004 and 0.02 mg/ml, more preferably between 0.008 to 0.012 mg/ml and/or polyethylene glycol in in a concentration comprised between 6 and 14 mg/ml, more preferably between 8 and 13 mg/ml, even more preferably between 9 and 12 mg/ml, even more preferably between 10 and 11 mg/ml.

11. Ophthalmic formulation as claimed in claims 1 to 10 for use in the prevention and/or treatment of corneal epithelial defects, preferably wherein said corneal epithelial defects are associated to impaired corneal sensitivity, dry eye, neurotrophic keratitis or surgery

## Patentansprüche

1. Ophthalmische Formulierung mit einer Zusammensetzung, die aus Trehalose, Mannitol, Hydroxypropylmethylcellulose und gegebenenfalls einer oder mehreren weiteren Komponenten besteht, die aus der Liste ausgewählt sind, die aus antioxidativen Verbindungen, Puffermitteln, Polyethylenglykolen und ophthalmisch verträglichen Hilfsstoffen besteht, wobei die ophthalmische Formulierung kein nichtionisches Tensid enthält.

2. Ophthalmische Formulierung nach Anspruch 1 mit einer Zusammensetzung, die aus Trehalose, Mannitol, Hydroxypropylmethylcellulose und gegebenenfalls einer oder mehreren weiteren Komponenten besteht, die aus der Liste ausgewählt sind, die aus antioxidativen Verbindungen, Puffermitteln und Polyethylenglykolen besteht.

3. Ophthalmische Formulierung nach Anspruch 1 oder 2, mit einer Zusammensetzung, die aus Trehalose, Mannitol, Hydroxypropylmethylcellulose, Puffermitteln und einem Polyethylenglykol besteht.

4. Ophthalmische Formulierung nach Anspruch 1 oder 2, wobei die antioxidativen Verbindungen L-Methionin und Cystein sind.

5. Ophthalmische Formulierung nach einem der Ansprüche 1 bis 4, wobei die Polyethylenglykole ein Molekulargewicht zwischen 4000 und 8000 g/mol, bevorzugter zwischen 5000 und 7000 g/mol, bevorzugter zwischen 5500 und 6500 g/mol, noch bevorzugter von 6000 g/mol aufweisen und/oder die Puffermittel ausgewählt sind aus Natriumphosphat, Phosphat/Citrat, Trisphosphat/Borat, Aminosäuremitteln oder Kombinationen davon, vorzugsweise Natriumphosphatpuffer.

6. Ophthalmische Formulierung nach einem der Ansprüche 1 bis 5 mit einer Zusammensetzung, bestehend aus Trehalose in einer Menge zwischen 60 und 85 Gew.-%, vorzugsweise zwischen 70 und 80 Gew.-%, Mannitol in einer Menge zwischen 10 und 30 Gew.-%, vorzugsweise zwischen 15 und 25 Gew.-%, Hydroxypropylmethylcellulose in einer Menge zwischen 0,5 und 2,5 Gew.-%, vorzugsweise zwischen 1 und 2 Gew.-% und gegebenenfalls Phosphatpuffer in einer Menge zwischen 3 und 10 Gew.-%, vorzugsweise zwischen 5 und 8 Gew.-%, Methionin in einer Menge zwischen 0,005 und 0,02 Gew.-%, vorzugsweise zwischen 0,01 und 0,015 Gew.-%, Polyethylenglykol in einer Menge zwischen 5 und 25 Gew.-%, vorzugsweise zwischen 10 und 20 Gew.-%.

7. Ophthalmische Formulierung nach einem der Ansprüche 1 bis 6, in Form eines Pulvers, vorzugsweise eines lyophilisierten Pulvers.

8. Ophthalmische Formulierung nach einem der Ansprüche 1 bis 6, in Form einer Lösung.

9. Ophthalmische Formulierung nach Anspruch 8, wobei der pH-Wert der Lösung vorzugsweise zwischen 6,5 und 8, bevorzugter zwischen 6,8 und 7,5, noch bevorzugter zwischen 7 und 7,4 liegt und/oder die Osmolarität der Lösung zwischen 200 und 380 mOsmol/kg, vorzugsweise zwischen 250 und 330 mOsmol/kg, bevorzugter zwischen 280 und 320 mosm/kg liegt.

10. Ophthalmische Formulierung nach Anspruch 8 oder 9, enthaltend Trehalose in einer Konzentration zwischen 40 und 60 mg/ml, bevorzugter zwischen 45 und 55 mg/ml, noch bevorzugter zwischen 48 und 52 mg/ml, noch bevorzugter zwischen 50 und 51 mg/ml, und/oder Mannitol in einer Konzentration zwischen 8 und 20 mg/ml, bevorzugter zwischen 10 und 16 mg/ml, noch bevorzugter zwischen 12 und 14 mg/ml, noch bevorzugter zwischen 13 und 13,5 mg/ml, und/oder Hydroxypropylmethylcellulose in einer Konzentration zwischen 0,4 und 2 mg/ml, bevorzugt zwischen 0,8 und 1,6 mg/ml, noch bevorzugter zwischen 1 und 1,3 mg/ml, und/oder ein Puffermittel in einer Konzentration zwischen 2 und 6 mg/ml, bevorzugt zwischen 4 und 5 mg/ml, und/oder ein Antioxidans in einer Konzentration zwischen 0,004 bis 0,02 mg/ml, bevorzugter zwischen 0,008 bis 0,012 mg/ml und/oder Polyethylenglykol in einer Konzentration zwischen 6 und 14 mg/ml, bevorzugter zwischen 8 und 13 mg/ml, noch bevorzugter zwischen 9 und 12 mg/ml, noch bevorzugter zwischen 10 und 11 mg/ml.

11. Ophthalmische Formulierung nach einem der Ansprüche 1 bis 10 zur Verwendung bei der Vorbeugung und/oder Behandlung von Hornhautepitheldefekten, wobei die Hornhautepitheldefekte vorzugsweise mit einer beeinträchtigten Hornhautempfindlichkeit, einem trockenen Auge, einer neurotrophen Keratitis oder einer Operation assoziiert sind.

## Revendications

1. Formulation ophtalmique ayant une composition constituée de tréhalose, de mannitol, d'hydroxypropylméthyl cellulose et, éventuellement, d'un ou plusieurs autres composants choisis dans la liste constituée de composés antioxydants, d'agents tampons, de polyéthylène glycols et d'excipients acceptables du point de vue ophtalmique, dans laquelle la formulation ophtalmique ne comprend pas de tensioactif non ionique.

2. Formulation ophtalmique selon la revendication 1, ayant une composition constituée de tréhalose, de mannitol, d'hydroxypropylméthyl cellulose et, éventuellement, d'un ou plusieurs autres composants choisis dans la liste constituée de composés antioxydants, d'agents tampons et de polyéthylène glycols.

3. Formulation ophtalmique selon les revendications 1 ou 2, ayant une composition constituée de tréhalose, de mannitol, d'hydroxypropylméthyl cellulose, d'agents tampons et d'un polyéthylène glycol.

4. Formulation ophtalmique selon la revendication 1 ou 2, dans laquelle lesdits composés antioxydants sont la L-méthionine et la cystéine.

5. Formulation ophtalmique selon les revendications 1 à 4, dans laquelle lesdits polyéthylène glycols ont un poids moléculaire compris entre 4000 et 8000 g/mol, de préférence entre 5000 et 7000 g/mol, de préférence entre 5500 et 6500 g/mol, plus de préférence encore de 6000 g/mol et/ou lesdits agents tampons sont choisis parmi le phosphate de sodium, le phosphate/citrate, le tris phosphate/borate, les agents d'acides aminées ou des combinaisons de ceux-ci, de préférence le tampon phosphate de sodium.

6. Formulation ophtalmique selon les revendications 1 à 5, ayant une composition constituée de tréhalose en une quantité comprise entre 60 et 85 % en poids, de préférence entre 70 et 80 % en poids, de mannitol en une quantité comprise entre 10 et 30 % en poids, de préférence entre 15 et 25 % en poids, d'hydroxypropylméthyl cellulose en une quantité comprise entre 0,5 et 2.5 % en poids, de préférence entre 1 et 2 % en poids et, éventuellement, un tampon phosphate en une quantité comprise entre 3 et 10 % en poids, de préférence entre 5 et 8 % en poids, de la méthionine en une quantité comprise entre 0,005 et 0,02 % en poids, de préférence entre 0,01 et 0,015 % en poids, du polyéthylène glycol en une quantité comprise entre 5 et 25 % en poids, de préférence entre 10 et 20 % en poids.

7. Formulation ophtalmique selon les revendications 1 à 6, sous forme de poudre, de préférence lyophilisée.

8. Formulation ophtalmique selon les revendications 1 à 6, sous forme d'une solution.

9. Formulation ophtalmique selon la revendication 8, dans laquelle le pH de ladite solution est de préférence compris entre 6,5 et 8, de préférence encore entre 6,8 et 7,5, plus de préférence encore entre 7 et 7,4 et/ou l'osmolarité de ladite solution est comprise entre 200 et 380 mOsmol/kg, de préférence entre 250 et 330 mOsmol/kg, de préférence encore entre 280 et 320 mOsm/Kg.

10. Formulation ophtalmique selon la revendication 8 ou 9, contenant du tréhalose à une concentration comprise entre 40 et 60 mg/ml, de préférence entre 45 et 55 mg/ml, plus de préférence encore entre 48 et 52 mg/ml, plus de préférence encore entre 50 et 51 mg/ml, et/ou du mannitol à une concentration comprise entre 8 et 20 mg/ml, de préférence entre 10 et 16 mg/ml, plus de préférence encore entre 12 et 14 mg/ml, plus de préférence encore entre 13 et 13,5 mg/ml et/ou de l'hydroxypropylméthyl cellulose à une concentration comprise entre 0,4 et 2 mg/ml, de préférence entre 0,8 et 1,6 mg/ml, plus de préférence encore entre 1 et 1,3 mg/ml, et/ou un agent tampon à une concentration comprise entre 2 et 6 mg/ml, de préférence entre 4 et 5 mg/ml, et/ou un antioxydant à une concentration comprise entre 0,004 à 0,02 mg/ml, de préférence encore entre 0,008 à 0,012 mg/ml et/ou du polyéthylène glycol à une concentration comprise entre 6 et 14 mg/ml, de préférence encore entre 8 et 13 mg/ml, plus de préférence encore entre 9 et 12 mg/ml, plus de préférence encore entre 10 et 11 mg/ml.

11. Formulation ophtalmique selon les revendications 1 à 10 pour la prévention et/ou le traitement des défauts de l'épithélium cornéen, de préférence lorsque lesdits défauts de l'épithélium cornéen sont associés à une altération de la sensibilité cornéenne, à la sécheresse oculaire, à la kératite neurotrophique ou à une intervention chirurgicale.
